# EUROPEAN PATENT SPECIFICATION

(11) **EP 0 463 887 B1**
(45) Date of publication and mention of the grant of the patent: **06.12.1995**
(21) Application number: 91305933.3
(22) Date of filing: 01.07.1991
(51) Int. Cl.: A61L 33/00

(54) **Collagen fiber hemostatic material and method of producing the same**
Hämostatisches Material aus Kollagenfasern und Verfahren zu seiner Herstellung
Matériau hémostatique en fibres de collagène et sa méthode de production

(30) Priority: 29.06.1990 JP 169866/90
(43) Date of publication of application: 02.01.1992
(73) Proprietor: Koken Company Limited, Shinjuku-ku Tokyo (JP)
(72) Inventor: Kuru,Satoshi c/o Koken Bioscience Inst., Meguro-ku, Tokyo (JP); Miyata,Teruo c/o Koken Bioscience Inst., Meguro-ku, Tokyo (JP); Sato,Yukihiro c/o Koken Tsuruoka Factory, Tsuruoka-shi, Yamagata-ken (JP)
(74) Representative: Gore, Peter Manson

(56) References cited:
- EP-A- 0 092 414
- EP-A- 0 212 881
- EP-A- 0 212 933
- EP-A- 0 297 972
- DE-A- 2 716 602
- US-A- 4 233 360
- US-A- 4 271 070

## Description

The present invention relates to collagen fiber hemostatic material which can be used for stopping bleeding caused by operation, injury or the like and a process for preparing the same.

Absorptive hemostatic materials have heretofore been used to stop bleeding from organs at the time of surgical operations, from vascular junctions or due to injury. Absorptive hemostatic materials of fibrous materials in cottony form have been often used because of their excellent operability. Cellulose oxide fiber in cottony form has been used, but it has a defect in that its absorbance into a living body is delayed due to the fact that it is not a substance which originates from a living body. Collagen fiber in cottony form having platelet aggregation by itself has been noted as a possible hemostatic material. This material is made by cutting insoluble collagen into cottony form, but this insoluble collagen is difficult to purify, whereby and thus it was difficult to obtain a product free of impurities.

There has recently been published a process which comprises spinning soluble collagen into cotton-like fibrous form and using the resulting material as hemostatic material. This process comprises hemogenizing pig skin, treating it with pepsin to provide a collagen solution, spinning it into a coagulating bath to provide a fiber 50 »m in average diameter, cutting the fiber into lengths of 2 to 5 cm, desalting with methanol, dehydrating and drying the resulting collagen. See JINKO ZOKI (Artificial Organs), Vol. 19,No.3(1990), 1235 to 1238. This cottony hemostatic material has good properties because it can be purified and shows good absorption into a living body. It has a defect in that the surface of the fiber is comparatively smooth, and due to this defect its absorption of blood is not sufficient even when applied with pressure to the bleeding moiety, due in part to its insufficient affinity for the bleeding moiety. Accordingly, this cottony collagen does not provide a satisfactory hemostatic effect.

An object of the present invention is to provide a collagen fiber hemastatic material and a process for preparing the same.

Another object ot the present invention is to provide a hemostatic material which contains cotton-like material or felt-like material of a fiber which is made by cross-linking the collagen molecule of soluble collagen fiber, where the fiber has a thickness of 10 to 40 »m and fine crack-like fissures on its surface.

One embodiment of the invention will now be described, by way of example, with reference to Figures 1, 2 and 3.

Figure 1 is a microscopic photograph of the lyophilized, cross-linked collagen fiber of the hemostatic material of the present invention.

Figures 2 and 3 are microscopic photographs similar to that of Figure 1 at larger magnifications.

A fiber usable in the hemostatic material of the present invention can be a fiber having very fine crack-like fissures on its surface made by cross-linking the collagen molecule of soluble collagen fiber (hereinafter referred to as "cross-linked collagen fiber").

The very fine crack-like fissures on the surface of the cross-linked collagen fiber usable in the hemostatic material of the present invention greatly increases its surface area, so that it can absorb blood as a whole. When the hemostatic material of the present invention is placed against a bleeding region in a body, it markedly accelerates the rate of blood coagulation because it has a good affinity with the bleeding moiety, region or part of a living body. Further, the workability or adaptability of the hemostatic material of the present invention for placement on the bleeding region can be improved by using fibers having a thickness of 10 to 40 »m to make the hemostatic material.

The cross-linked collagen fiber is preferably used in a cotton-like form. This can, for example, take the shape of a cotton ball. This cottony shape of the hemostatic material permits its shape to be freely changed together with easy adjustment of the amount used, making it applicable to virtually any shaped bleeding region. In addition to a cotton-like form, the cross-linked collagen fiber of the present invention can also be constructed in another desired form such as a gauze, felt or non-woven fabric. The felt form can be prepared by crushing and/or adhering the cotton-like form to another fiber so as to provide a felt form.

The soluble collagen used to prepare the fibers of the present invention can include atelocollagen and atelocollagen derivatives which can be made by esterifying the carboxy group of the collagen molecule. The soluble collagen such as atelocollagen or the like usable as raw material in the present invention can be easily purified to give goodnatured fiber. The soluble collagen fiber of the present invention becomes washable with water by cross-linking the spun fiber with glutaraldehyde, epoxy compounds or similar materials conventionally used for cross-linking the collagen molecule. The living body-absorbing rate of the collagen fiber can be controlled by varying the amount or degree of cross-linking. The rate of cross-linking which is measured as the rate of reaction of ε-amino groups on the side chains in collagen normally ranges from about 1% to about 50% or less, more preferably up to about 30%. A higher amount of cross-linking will slow the body-absorbing rate, and a lower amount of cross-linking will increase the body-absorbing rate. Accordingly, the live body-absorbing rate of the hemostatic material of the present invention can be conveniently adjusted, depending upon the conditions of the bleeding region where it is to be used.

Alter spinning, the fiber may be allowed to react with protamine to form a complex, which can be washed and lyophilized. In this case, the complex is made by reacting protamine with collagen via a difunctional cross-linking agent such as glutaraldehyde, hexamethylene diisocyanate or the like.

The cross-linked collagen fiber of the present invention can be prepared, for example, as described below.

First atelocollagen is extracted by treating bovine derma or the like with an enzyme. This atelocollagen is purified by subjecting it to depyrogen, dealbumin or the like treatment. Because atelocollagen is soluble, it can easily be purified. A spinning solution is prepared from such purified atelocollagen. An appropriate collagen concentration in the spinning solution can be 2 to 20%. The soluble collagen fiber is then made by spinning this spinning solution into an appropriate coagulation bath. An aqueous solution of sodium chloride, sodium sulfate or the like can be used for the coagulation solution.

The resulting spun soluble collagen fiber is treated with a conventional cross-linking agent such as glutaraldehyde, epoxy compounds or the like typically used for cross-linking the collagen molecule. The cross-linking treatment can be effected either after once drying the spun soluble collagen fiber or on fibers in the wet state shortly after spinning. Further, the cross-linking may be effected in acidic or alkaline conditions.

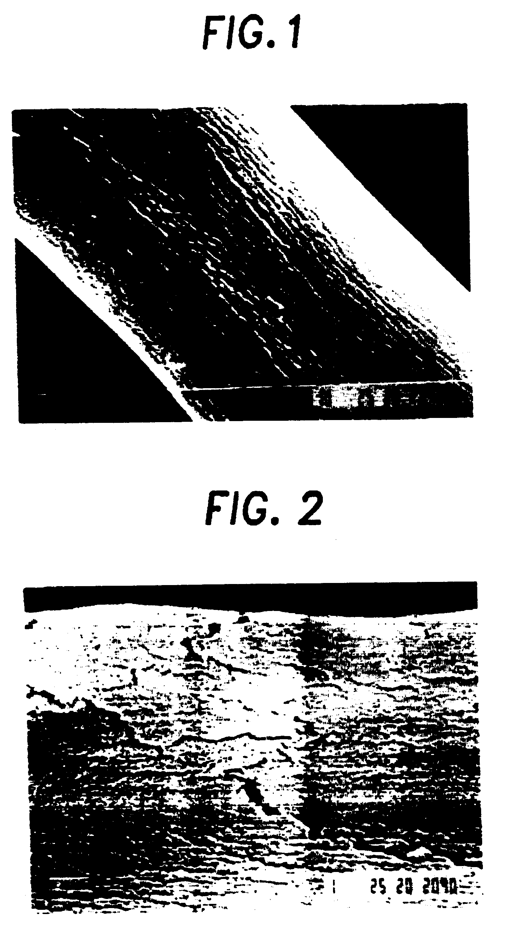

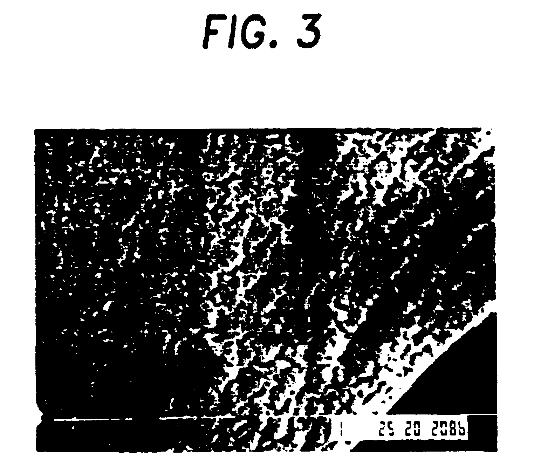

After washing, the fiber is lyophilized (freeze-dried). During this lyophilization, the surface of cross-linked collagen fiber becomes cracked, so as to have numerous very fine fissures contained therein, as shown in Figures 1 to 3. As shown in Figures 1 to 3, the fissures or cracks in the surface of the fiber can vary greatly from, for example, very fine cracks of about 0.1 »m in width up to fine cracks of about 0.5 »m in thickness. In Figure 1 the white bar represents 10 »m in length, and in Figures 2 and 3 the white bar represents 1 »m in length. Conventional freeze-drying techniques can be used to lyophilize the cross-linked collagen fiber. Where the fiber is frozen the frozen fiber is dried in a vacuum so that during the drying process the fiber does not melt. The lyophilized cross-linked collagen fiber has a surface area which is markedly increased in comparison with the soluble collagen dried by dehydration after spinning. The hemostatic material made from the freeze-dried cross-linked collagen fiber of the present invention has a greatly improved blood absorption and affinity with the bleeding region. Figure 1 is a microscopic photograph of the surface of the freeze-dried cross-linked collagen fiber described above at a magnification of about 7000. Figures 2 and 3 are microscopic photographs of a further enlarged surface of this fiber respectively at magnifications of 10,000 and 20,000.

Lyophilization can be performed on wet fibers containing a substantial amount of water, e.g. fibres wet from washing with water, or can be performed on fibers after dehydrating with a centrifuge or the like. In the former case, the lyophilized fiber takes a cotton-like form which does not need to be subjected to a disentangling treatment, but in the latter case, the lyophilized fiber usually needs to be disentangled so as to provide the cotton-like form. Disentanglement can be achieved by conventional methods and machines which unravels fibers adhered or entangled together to form fine fibers.

After lyophilizing, the cross-linked collagen fiber can be shaped into cotton-like, felt-like or other appropriate form; sterilized with, for example, γ-ray, ethylene oxide gas or the like and then packed, for example, in sterilized packaging before provided to users.

Furthermore, the pH of the hemostatic material of the present invention can be used in neutral or acidic, but an acidic medium is preferred in view of its higher hemostatic effect.

The following Examples are illustrative of the present invention:

### Example 1

Fresh bovine derma was pulverized very finely, and the resulting very fine powder was washed repeatedly with 0.1 M aqueous sodium acetate and then washed with water. After washing with water, the very fine powder was extracted with 0.5 M aqueous acetic acid, and the residual insoluble was filtered. This insoluble collagen (100 g) was recover in wet form, mixed with 1ℓ of 0.5 M acetic acid and then 0.1 g of pepsin and stirred at 20°C for 3 days. This treatment transformed the insoluble collagen into viscous soluble collagen (namely, atelocollagen) solution. This atelocollagen solution was filtered, and the filtrate was adjusted to a pH of 7.5 to give a precipitate. The precipitate was separated by a centrifuge and washed three times with water to give atelocollagen.

This atelocollagen was made into 3% acidic solution which was extruded from a nozzle having 200 pores of 70 »m diameter into saturated aqueous sodium sulfate solution. The resulting threads or fibers were dipped in dilute hydrochloric acid having a pH of 4 and containing 0.5% glutaraldehyde (and containing 15% NaCℓ) for 15 minutes and washed with water. After washing with water, the threads were cut into lengths of about 5 cm and lyophilized. After drying, the resulting threads had the form of a cotton-like hemostatic material without further modification. The hemostatic material was sterilized with γ-ray and used in Example 6 described below.

### Example 2

A plurality of cotton-like hemostatic about 1 cm thick as obtained in Example 1 were pressed to give felt-like cloth hemostatic material having a thickness of 3 cm.

### Example 3

The threads spun in the same manner as in Example 1 were dipped in a 1% solution of protamine sulfate and then in 1% glutaraldehyde solution for 10 minutes for cross-linking. The threads were washed with water and lyophilized as in Example 1 to give a cotton-like hemostatic material containing protamine. The hemostatic material was sterilized by γ-ray and used in Example 6 below.

### Example 4

The cotton-like hemostatic material obtained in Example 3 was pressed in the same manner as in Example 2, and a felt-like cloth hemostatic material was obtained.

### Example 5

The threads obtained by spinning in the same manner as in Example 1 were dipped in an aqueous weakly alkaline solution of pH 9 containing 0.5% glutaraldehyde for 10 minutes, and then dipped in a dilute hydrochloric acid solution having a pH of 3 for 1 hour. The threads were thoroughly washed with water and lyophilized in the same manner as in Example 1 to give a cotton-like hemostatic material.

### Example 6

The blood coagulating effect of the hemostatic materials obtained in Examples 1 and 3 were at first measured in vitro. The coagulating rate for each material was measured, where blood : hemostatic material = 5.0 mℓ : 0.1 g, namely 5.0mℓ of blood with 0.1 g of a respective hemostatic material. In this test the hemostatic material in Example 1 coagulated in about 30 seconds, and the hemostatic material in Example 3 coagulated in about 18 seconds. On the other hand with the same test, commercially available cellulose hemostatic material coagulated in about 90 seconds, and insoluble powdery collagen hemostatic material coagulated in about 40 seconds. These tests demonstrate the improved blood coagulating ability of the hemostatic material of the present invention.

Further, an animal test with a dog's spleen was carried out. In this test a dog's spleen was cut off with a knife and the amount of time required to stop the resulting bleeding with different hemostatic material was measured. In this test, the hemostatic material in Example 1 stopped the bleeding in 150 seconds, that of Example 3 stopped the bleeding in 175 seconds, while the commercially available cellulose hemostatic material required 310 seconds and in soluble powdery collagen hemostatic material needed 190 seconds. Thus it was evident that the hemostatic material of the present invention was very effective for stoppage of bleeding.

Since collagen fiber, namely the material coming from a living body, such as a mammal is used to prepare the hemostatic material of the present invention, the material of the present invention can provide an effective living body absorption rate without foreign reaction or rejection from the living body. Since the soluble collagen is used as a stating collagen for spinning, it can be easily purified to give a good-natured product. The surface of the fiber of the present invention is provided with very fine crack-like fissures which fissure increases its surface area and provides improved blood absorption. Further, the hemostatic material of the present invention shows an excellent affinity with bleeding regions so that blood coagulation will take place rapidly with a remarkable hemostatic effect. Still further, due to the fact that the hemostatic material of the present invention has a cotton-like or felt-like form made from fibers of 10 to 40 »m in thickness, it can be easily shaped into any form so as to adapt to any bleeding region in the living body.

In addition, the rate absorption of the hemostatic material of the present invention by the living body can be adjusted by varying the degree of cross-linking of the soluble collagen, where a higher amount of cross-linking slows absorption and a lower count of cross-linking speeds absorption. This enables the living body-absorption of the material of the present invention to be conveniently changed in accordance with the state of the bleeding region. Further, the hemostatic effect of the material of the present invention can be achieved even when the spun soluble collagen fiber is reacted with protamine to give a complex thereof, which can be ionically bonded with heparin.

An Additional advantage of the present invention is that the cross-linked collagen fibre having very fine crack-like fissures on its surface, can be easily prepared by simple methods such as spinning the soluble collagen, cross-linking, washing with water and lyophilizing. Accordingly, an improved hemostatic material can be prepared at a reasonable cost by the process of the present invention.

## Claims

1. A hemostatic material comprising cross-linked soluble collagen fibres, characterised in that said fibres have a thickness of 10 to 40 »m and a surface containing fine fissures.

2. A hemostatic material as claimed in claim 1 in which said fissures are from between 0.1»m and 0.5»m thick.

3. A hemostatic material as claimed in claim 1 or 2 wherein the cross-linked collagen fibre is complexed with protamine.

4. A hemostatic material as claimed in any of the preceding claims wherein the collagen fibre is cross-linked in an amount of about 1% to about 50% based on ε-amino groups on side chains of collagen.

5. A hemostatic material as claimed in claim 4 wherein the collagen fibre is cross-linked in an amount of about 1% to about 30% based on ε-amino groups on side chains of collagen.

6. A hemostatic material as claimed in any of the preceding claims further characterised in that it is in the form of a cotton-like material.

7. A hemostatic material as claimed in any of claims 1 to 5 further characterised in that it is in the form of a felt-like material.

8. A process for preparing collagen fibre for a hemostatic material comprising:
spinning an aqueous solution of soluble collagen into a coagulating bath,
cross-linking the resulting spun collagen fibre and washing it, said process being characterised in that the resulting cross-linked collagen fibre is lyophilized.

9. A process as claimed in claim 8 which further comprises reacting the spun collagen fibre with protamine to provide a complex therewith.

## Patentansprüche

1. Hämostatisches Material, das vernetzte lösliche Kollagenfasern umfaßt, dadurch gekennzeichnet, daß genannte Fasern eine Dicke von 10 bis 40 »m und eine Oberfläche mit feinen Fissuren haben.

2. Hämostatisches Material nach Anspruch 1, worin genannte Fissuren zwischen 0,1 »m und 0,5 »m dick sind.

3. Hämostatisches Material nach Anspruch 1 oder 2, worin die vernetzten Kollagenfasern mit Protamin im Komplex gebunden sind.

4. Hämostatisches Material nach einem der vorangehenden Ansprüche, worin die Kollagenfasern in einer Menge von circa 1% bis circa 50% bezogen auf ε-Aminogruppen an Seitenketten von Kollagen vernetzt sind.

5. Hämostatisches Material nach Anspruch 4, worin die Kollagenfasern in einer Menge von circa 1% bis circa 30% bezogen auf ε-Aminogruppen an Seitenketten von Kollagen vernetzt sind.

6. Hämostatisches Material nach einem der vorangehenden Ansprüche, darüber hinaus dadurch gekennzeichnet, daß es in der Form eines baumwollähnlichen Materials vorliegt.

7. Hämostatisches Material nach einem der Ansprüche 1 bis 5, darüber hinaus dadurch gekennzeichnet, daß es in der Form eines filzartigen Materials vorliegt.

8. Verfahren zur Herstellung von Kollagenfasern für ein hämostatisches Material, das folgendes umfaßt:
Spinnen einer wäßrigen Lösung aus löslichem Kollagen in einem Fällbad,
Vernetzen der sich ergebenden gesponnenen Kollagenfasern und Waschen derselben, wobei genanntes Verfahren dadurch gekennzeichnet ist, daß die sich ergebenden vernetzten Kollagenfasern lyophilisiert werden.

9. Verfahren nach Anspruch 8, welches darüber hinaus folgendes umfaßt: Reagieren der gesponnenen Kollagenfasern mit Protamin zur Bereitstellung eines Komplexes damit.

## Revendications

1. Un matériau hémostatique composé de fibres de collagène solubles réticulées, caractérisées en ce que lesdites fibres ont une épaisseur de 10 à 40 »m et une surface contenant de fines fissures.

2. Un matériau hémostatique tel que revendiqué par la revendication 1 dans lequel lesdites fissures sont d'une épaisseur variant de 0,1 »m et 0,5 »m.

3. Un matériau hémostatique tel que revendiqué dans la revendication 1 ou 2 dans lequel la fibre de collagène réticulée est associée à la protamine.

4. Un matériau hémostatique tel que revendiqué dans l'une quelconque des revendications précédentes dans lequel la fibre de collagène est réticulée avec une quantité d'environ 1% à 50% basée sur des groupes amino-ε sur les chaînes latérales du collagène.

5. Un matériau hémostatique tel que revendiqué dans la revendication 4 dans lequel la fibre de collagène est réticulée avec une quantité d'environ 1% à 30% basée sur des groupes amino-ε sur les chaînes latérales du collagène.

6. Un matériau hémostatique tel que revendiqué dans l'une quelconque des revendications précédentes caractérisé de plus en ce qu'il a la forme d'un matériau analogue à la ouate.

7. Un matériau hémostatique tel que revendiqué dans l'une quelconque des revendications 1 à 5 caractérisé de plus en ce qu'il a la forme d'un matériau analogue au feutre.

8. Un procédé pour préparer la fibre de collagène pour un matériau hémostatique comprenant les opérations suivantes :
le filage d'une solution aqueuse de collagène soluble dans un bain coagulant;
réticuler la fibre filée de collagène ainsi obtenue, la laver, ledit procédé étant caractérisé en ce que la fibre de collagène réticulée ainsi produite est lyophilisée.

9. Procédé tel que revendiqué à la revendication 8 par lequel on fait de plus réagir la fibre de collagène filée avec la protamine pour obtenir un produit complexe.
